Europäisches Patentamt
European Patent Office
Office européen des brevets

(19)

(11) Publication number: **0 379 737 A2**

(12) **EUROPEAN PATENT APPLICATION**

(21) Application number: **89124151.5**

(51) Int. Cl.5: **A61K 31/44, A61K 31/495**

(22) Date of filing: **29.12.89**

The title of the invention has been amended (Guidelines for Examination in the EPO, A-III, 7.3).

(30) Priority: **29.12.88 JP 333101/88**

(43) Date of publication of application: **01.08.90 Bulletin 90/31**

(84) Designated Contracting States: **BE CH DE ES FR GB IT LI NL SE**

(71) Applicant: **THE GREEN CROSS CORPORATION**
**3-3, Imabashi 1-chome Chuo-ku**
**Osaka-shi Osaka(JP)**

(72) Inventor: **Kido, Hideaki c/o The Green Cross Corporation**
**Central Res. Laboratories 2-1180-1, Shodai-ohtani**
**Hirataka-shi Osaka(JP)**
Inventor: **Uchida, Takeshi c/o The Green Cross Corporation**
**Central Res. Laboratories 2-1180-1, Shodai-ohtani**
**Hirataka-shi Osaka(JP)**
Inventor: **Fukaya, Chikara c/o The Green Cross Corporation**
**Central Res. Laboratories 2-1180-1, Shodai-ohtani**
**Hirataka-shi Osaka(JP)**
Inventor: **Yokoyama, Kazumasa c/o The Green Cross Corporation**
**Central Res. Laboratories 2-1180-1, Shodai-ohtani**
**Hirataka-shi Osaka(JP)**
Inventor: **Uchida, Yasumi c/o The Green Cross Corporation**
**Central Res. Laboratories 2-1180-1, Shodai-ohtani**
**Hirataka-shi Osaka(JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) **Dihydropyridine derivatives for the treatment of angiospasm.**

(57) A treating agent of angiospasm is disclosed, which contains, as an active ingredient, a dihydropyridine derivative represented by formula (I):

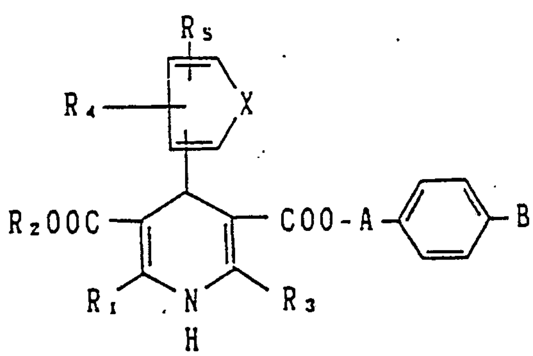

( I )

wherein $R_1$, $R_2$ and $R_3$ which may be the same or different, each represents an alkyl group or an alkoxyalkyl group; $R_4$ and $R_5$, which may be the same or different, each represents a hydrogen atom, a

EP 0 379 737 A2

halogen atom, a nitro group, a halogenated alkyl group, an alkyl-sulfonyl group, a halogenated alkoxyl group, an alkylsulfinyl group, an alkyl group, an alkoxyl group, a cyano group, an alkoxycarbonyl group or an alkylthio group, provided that $R_4$ and $R_5$ do not simultaneously represent a hydrogen atom; X represents a vinylene group or an azomethine group; A represents an alkylene group ; and B represents $-N(R_6)_2$ or

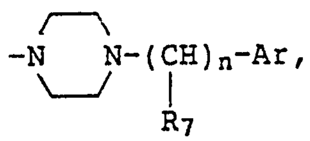

wherein $R_6$ and $R_7$ each represents a hydrogen atom, an alkyl group, an aralkyl group, an aryl group or a pyridyl group; Ar represents an aryl group or a pyridyl group; and n represents 0 or an integer of 1 or 2, or a pharmaceutically acceptable acid addition salt thereof. The agent exhibits high angiospasm inhibitory activity and extremely low toxicity.

# TREATING AGENT OF ANGIOSPASM

## FIELD OF THE INVENTION

This invention relates to an agent for treating angiospasm containing a dihydropyridine derivative as an active ingredient.

## BACKGROUND OF THE INVENTION

Angiospasm is transient tonic constriction of blood vessels, particularly arteries, and induces considerable functional disturbances of organs governed by the blood vessels due to interruption of blood flow.

Angiospasm frequently occurs in the coronary arteries, cerebral arteries, and arteries of limbs.

Angiospasm is caused by apoplexy, subarachnoid hemmorrhage, cerebral thrombosis, etc. in the brain; or endothelial lesions, arteriosclerosis, etc. in the coronary arteries and, as a result, induces cerebral ischemia, cerebral infarct, myocardial ischemia, myocardial infarction, angina pectoris, etc.

In the peripheral vessels, angiospasm causes a Raynaud phenomenon and intermittent claudication. When it occurs in arteries controlling digestive organs, it induces ischemic colitis, etc.

Conventionally employed treating agents of angiospasm include nitroglycerin and Nicorandil. On the other hand, dihydropyridine compounds have been considered useful for the treatment of angiospasm, and the use of some of them has been extending (cf. Acta Neurochir, 82, 110-114 (1986) and Radiation Medicine, 1, 299-304 (1983)).

A dihydropyridine derivative of this invention is described in EP-A-257616, but its angiospasm-inhibiting activity is not known.

## SUMMARY OF THE INVENTION

An object of this invention is to provide a treating agent for angiospasm exhibiting extremely excellent activity.

As a result of extensive investigations, the inventors have found that a dihydropyridine derivative represented by formula (I) shown below exhibits particularly excellent activity of inhibiting angiospasm.

This invention relates to a treating and/or preventing agent of angiospasm, and use thereof, containing, as an active ingredient, a dihydropyridine derivative represented by formula (I):

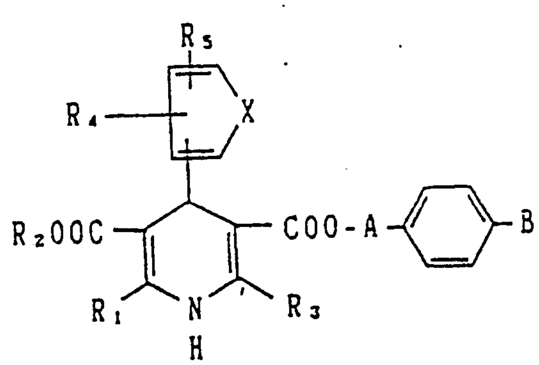

( I )

wherein $R_1$, $R_2$ and $R_3$ which may be the same or different, each represents an alkyl group or an alkoxyalkyl group; $R_4$ and $R_5$, which may be the same or different, each represents a hydrogen atom, a halogen atom, a nitro group, a halogenated alkyl group, an alkylsulfonyl group, a halogenated alkoxyl group, an alkylsulfinyl group, an alkyl group, an alkoxyl group, a cyano group, an alkoxycarbonyl group or an alkylthio group, provided that $R_4$ and $R_5$ do not simultaneously represent a hydrogen atom; X represents a vinylene group or an azomethine group; A represents an alkylene group; and B represents $-N(R_6)_2$ or

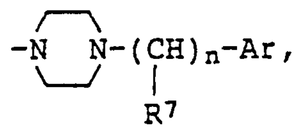

wherein $R_6$ and $R_7$ each represents a hydrogen atom, an alkyl group, an aralkyl group, an aryl group or a pyridyl group; Ar represents an aryl group or a pyridyl group; and n represents 0 or an integer of 1 or 2, or a pharmaceutically acceptable acid addition salt thereof.

## DETAILED DESCRIPTION OF THE INVENTION

The dihydropyridine derivative represented by formula (I) and an acid addition salt thereof according to the present invention have a specific structure unlike conventionally known dihydropyridine compounds and exhibit particularly excellent activity ascribed to this structural specificity. That is, they have high selectivity to organs or tissues in manifestation of their vasodilatory activity and exremely low toxicity and are therefore characterized by high effectiveness and safety.

In formula (I), the alkyl group as represented by $R_1$, $R_2$, or $R_3$ may have a straight or branched chain and, preferably, includes lower alkyl groups having from 1 to 6 carbon atoms, e.g., methyl, ethyl, propyl, isopropyl, butyl, isobutyl, sec-butyl, t-butyl, pentyl, isopentyl, neopentyl and hexyl groups. Particularly preferred of them are those having from 1 to 4 carbon atoms. These alkyl groups may have a lower cycloalkyl group having from 3 to 6 carbon atoms at the terminal thereof (e.g., cyclopropylmethyl, cyclobutyl ethyl and cyclopentylmethyl groups).

Also, the alkyl group can be a cycloalkyl group. The cycloalkyl group as represented by $R_1$, $R_2$ or $R_3$ preferably includes lower cycloalkyl groups having from 3 to 6 carbon atoms, e.g., cyclopropyl, cyclobutyl, cyclopentyl and cyclohexyl groups.

The alkoxyalkyl group as represented by $R_1$, $R_2$ or $R_3$ preferably includes those having from 3 to 7 carbon atoms in total, e.g., methoxyethyl, ethoxyethyl, propoxyethyl, isopropoxyethyl, butoxyethyl, methoxypropyl, 2-methoxy-1-methylethyl, and 2-ethoxy-1-methylethyl groups.

The substituents represented by $R_4$ and $R_5$, which may be the same or different, may be bonded at any position on the ring but preferably at the 2- and/or 3-position with respect to the dihydropyridine ring.

The halogen atom as represented by $R_4$ or $R_5$ includes fluorine, chlorine, bromine and iodine atoms, with fluorine or chlorine atom being preferred. The alkyl or cycloalkyl group preferably includes those enumerated for $R_1$, $R_2$ or $R_3$. The alkoxyl or alkylthio group preferably includes those having a lower alkyl group containing from 1 to 3 carbon atoms in the alkyl moiety thereof, e.g., methoxy, ethoxy, propoxy, isopropoxy, methylthio, ethylthio, propylthio and isopropylthio groups. The alkoxycarbonyl group includes those having from 2 to 4 carbon atoms, e.g., methoxycarbonyl and ethoxycarbonyl groups. The halogen atom in the halogenated alkyl or alkoxyl group includes those described above. Implicit in the halogenated alkyl group are those having a part of their hydrogen atoms halogenated [e.g., $(CF_3)_2CHCH_2$- and $CF_3CH_2$-] and those having all of their hydrogen atoms halogenated (e.g., trifluoromethyl group). The halogenated alkoxyl group also includes those having a part of their hydrogen atoms halogenated and those having all of their hydrogen atoms halogenated. The alkyl moiety in the alkylsulfonyl or alkylsulfinyl group includes those recited above for $R_1$, $R_2$ or $R_3$.

$R_4$ or $R_5$ preferably represents a cyano group or a halogenated alkyl group (particularly trifluoromethyl).

The alkyl group as represented by $R_6$ or $R_7$ includes those enumerated above for $R_1$, $R_2$ and $R_3$ including cycloalkyl. The aralkyl group includes those having an alkyl group containing 1 to 3 carbon atoms, e.g., benzyl, $\alpha$-phenylethyl, $\beta$-phenylethyl, and $\gamma$-phenylpropyl groups. The aryl group of aralkyl and aryl includes phenyl and naphthyl groups. These aromatic rings may have one or more substituents, which may be the same or different, at an arbitrary position thereof. Examples of the substituents include those enumerated above for $R_4$ or $R_5$. The pyridyl group includes 2-pyridyl, 3-pyridyl and 4-pyridyl groups, each of which may have one or more of sustituents as described for $R_4$ or $R_5$.

The alkylene group as represented by A preferably includes those having from 2 to 4 carbon atoms, either straight or branched, e.g., ethylene, trimethylene, tetramethylene and 1,2-dimethylethylene groups.

The aryl or pyridyl group as represented by Ar include those described above for $R_6$ and $R_7$ and may have one or more substituents described for $R_4$ or $R_5$.

The ring

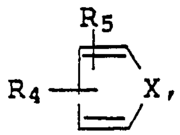

a substituent at the 4-position of the dihydropyridine ring, includes a benzene ring with X being a vinylene group (-CH=CH-) and a pyridine ring with X being an azomethine group (-CH=N-), each of which is bonded to the 4-position of the dihydropyridine ring at any position thereof.

The substituent $R_4$ or $R_5$ is bonded at any of ortho-, meta-, and para-positions, preferably at the ortho- and/or meta-position(s), with respect to the carbon atom at the 4-position of the dihydropyridine ring.

Specific examples of the dihydropyridine derivatives represented by formula (I) and acid addition salts thereof are 2-[p-(4-benzhydrylpiperazino)phenyl]-ethyl methyl 2,6-dimethyl-4(3-nitrophenyl)-1,4-dihydropyridine-3,5-dicarboxylate, 2-[p-(4-benzhydrylpiperazino)-phenyl]ethyl methyl 2,6-dimethyl-4-(4-cyano-2-pyridyl)-1,4-dihydropyridine-3,5-dicarboxylate, etc.; and acid addition salts thereof.

The dihydropyridine derivative of formula (I) can be prepared in a conventional manner, wherein for example, moieties for constituting the desired compound of formula (I) are cyclized by dehydration.

More specifically, processes described in U.S. Patent 4,849,429 or EP-A-216542, EP-A-257616, EP-A-265947, EP-A-94159, JP-A-63-99042, JP-A-63-152351, and JP-B-61-260064 (the terms "JP-A" and "JP-B" as used herein mean an "unexamined published Japanese patent application" and an "examined published Japanese patent application", respectively) can be referred to.

The thus prepared dihydropyridine derivative of formula (I) can be isolated so as to have any purity as desired by appropriate combinations of known separation and purification means, such as concentration, extraction, chromatography, reprecipitation, and recrystallization.

Having a basic group, the dihydropyridine derivative of formula (I) can be converted to its acid addition salt by known techniques. The acid addition salt which can be used is not particularly limited as long as it is of low toxicity as pharmacologically accepted and includes salts with inorganic acids (e.g., hydrochloride, hydrobromide, phosphate, and sulfate) and salts with organic acids (e.g., acetate, succinate, maleate, fumarate, malate, and tartarate).

The dihydropyridine derivative of formula (I) and acid addition salts thereof are of extremely low toxicity and exhibit potential and long-lasting effects to reduce angiospasm in mammals (e.g., mice, rats, rabbits, dogs, cats, and humans).

Accordingly, the dihydropyridine derivative of formula (I) or an acid addition salt thereof is useful for prevention and treatment of angiospasm.

The angiospasm treating agent according to the present invention is useful for prevention or treatment of angiospasm in the cerebral vessels (e.g., cerebral angiospasm following apoplexy or subarachnoid hemorrhage), the coronary vessels [including an attack of spasm which is developed when the site of stenosis is opened by laser cauterization, etc. and, particularly, angiospasm which is difficult to treat and resistant to nitroglycerin, i.e., a postoperative attack of coronarism and stenocardia induced by coronarism (coronary spasmodic stenocardia)] and other blood vessels.

In using the dihydropyridine derivative of formula (I) or an acid additions salt thereof as an active ingredient of the above-described drug, it is mixed with pharmaceutically necessary ingredients such as pharmacologically acceptable additives (e.g., carriers, vehicles, diluents, etc.) and formulated into pharmaceutical dose forms such as powders, granules, tablets, capsules, and injectable solutions. The drug can be administered orally or non-orally. An effective dose of the dihydropyridine derivative of formula (I) or an acid addtion salt thereof is compounded into the preparations. While the dose varies depending on the administration route, the body weight and age of a patient and other factors, it is desirable in, for example, oral administration to an adult to administer 0.05 to 20 mg/kg-b.w./day, particularly 0.1 to 4 mg/kg-b.w./day, in a single or several divided doses per day. In parenteral administration, it is desirable to intravenously administer 0.1 to 300 µg/kg-b.w./day, particularly 5 to 100 µg/kg-b.w./day, in a single or several divided doses per day (b.w. means "body weight").

The present invention is now illustrated in greater detail with reference to the following Test Examples, Reference Example, and Formulation Examples, but it should be understood that the present invention is not construed as being limited thereto.

Unless otherwise specified, determinations of $^1$H-NMR spectra in these examples were conducted using $CDC\ell_3$.

## TEST EXAMPLE 1

### Toxicity

$LD_{50}$ was determined for Compound 2 as prepared in Reference Example 1 below using 10 to 11-week-old male mice (body weight: 14 to 16 g; 3 to 5 mice per group). AS a result, measured $LD_{50}$ values were more than 1,100 mg/kg at the lowest. Most of the test compounds had an $LD_{50}$ of more than 1,400 mg/kg.

The compounds according to the present invention thus proved to have significantly lower acute toxicity and higher safety as compared with known compounds.

## TEST EXAMPLE 2

Preventive effect of premedication of coronary vasodilators on coronarism induced by laser cauterization of the coronary artery was examined using dogs.

Twenty-three anesthetized dogs premediated with a coronary vasodilator [8 dogs in non-treated group (control group); 7 in nitroglycerin-treated (TNG) group (100 µg i.c. (intracoronary) + 50 µg/min d.i.v. (intravenous drip injection)); 5 in Nicorandil (NC) group (30 µg/kg/min d.i.v); and 8 in Compound 2 (hereinafter described) group (10 µg/kg i.v.)] were used. A 9F guiding catheter was inserted from the right common carotid artery into the 28th circumflex ramus extending from the anterior descending branch of the right common carotid artery. After intravenous injection of 250 U of heparin and 20 mg of lidocaine, a probe having an argon laser heating tip having a diameter of 1.5 mm (manufactured by Trimedyne Corp.) was inserted through the catheter, and 10 minutes after the administration of drugs the coronary vessel was cauterized under a condition of 4.5 W × 30 minutes under photofluoroscopic observation. Before the cauterization, immediately after the cauterization, and 10 minutes and 30 minutes after the cauterization, changes of the coronary artery were angiographically observed. The degree of stenosis (%) was calculated from the measured inner diameter each of the cauterized site and the normal site. The degree of stenosis of a site undergoing retardation of angiography was taken as 99%.

The results obtained are shown in Table 1 below.

TABLE 1

| Group | Degree of Stenosis (%) (m ± SE) | | |
|---|---|---|---|
| | Immediately After | 10 mins. After | 30 mins. After |
| Control (n = 8) | 87 ± 6 | 82 ± 9 | 80 ± 11 |
| TNG (n = 7) | 71 ± 4 | 73 ± 17 | 66 ± 17 |
| NC (n = 5) | 38 ± 11** | 28 ± 9** | 29 ± 8** |
| Compound 2 (n = 8) | 12 ± 12 | 9 ± 10** | 12 ± 12** |

Note: **: $p < 0.01$

## REFERENCE EXAMPLE 1

Sythesis of 2-[p-(4-benzhydrylpiperazino)phenyl]ethyl methyl 2,6-dimethyl-4-(3-nitrophenyl)-1,4-dihydropyridine-3,5-dicarboxylate (Compound 1) and its hydrochloride (Compound 2):

In 100 mℓ-volume egg-plant type flask equipped with a Dimroth condenser were charged 1.144 g (7.57

mmol) of 3-nitrobenzaldehyde, 3.464 g (7.59 mmol) of [p-(4-benzhydrylpiperazino)phenyl]ethyl acetacetate, and 873 mg (7.58 mmol) of methyl 3-aminocrotonate, and 12 mℓ of isopropanol was added thereto. The mixture was heat-refluxed for 16 hours. The solvent was removed by distillation under reduced pressure, and the residue was subjected to silica gel column chromatography using chloroform-methanol (45:1 by volume) as an eluent followed by silica gel column chromatography using ethyl acetate-n-hexane (2:3 by volume) as an eluent. The resulting crude product was purified by high performance liquid chromatography to obtain 2.503 g (yield 48%) of the entitled Compound 1.

$IR\nu_{max}^{KBr}$ $cm^{-1}$ : 1680(C=0), 1520 ($NO_2$)

$^1$H-NMR δ : 8.06 (1H, t, J=2Hz), 7.97 (1H, ddd, J=8; 2; 1Hz), 7.1-7.6 (12H), 7.03 (2H, d, J=8.6Hz), 6.80 (2H, d, J=8.6Hz), 6.02 (1H, s), 5.07 (1H, s), 4.26 (1H, s), 4.22 (2H, t, J=7Hz), 3.64 (3H, s), 3.15 (4H, dd, J=5; 4.7Hz), 2.81 (2H, d, J=7Hz), 2.55 (4H, dd, J=5; 4.7Hz), 2.33 and 2.28 (each 3H, s).

In a 200 mℓ-volume egg-plant type flask was put 2.124 g (3.16 mmol) of Compound 1, and a rubber septum was fitted to the flask. In the flask was charged 100 mℓ of methylene chloride to dissolve the content, followed by stirring for 30 minutes at room temperature while introducing hydrogen chloride to the solution. The precipitated crystals were collected by filtration to obtain about 2.22 g of the entitled Compound 2.

$IR\nu_{max}^{KBr}$ $cm^{-1}$ : 2450 ($\rightarrow N^+H^-C\ell$), 1680 (C=0), 1525 ($NO_2$), 1350 ($NO_2$), (2514-07).

$^1$H-NMR δ : 13.72 (1H, brs.), 8.05-7.9 (6H), 7.82, 7.26 (4H, $A_2B_2$.q, J=8.6Hz), 7.6-7.3 (8H), 6.28 (1H, s), 5.2-5.05 (2H), 5.01 (2H, s), 4.27 (2H, t, J=6.5Hz), 4.3-4.1 (2H), 3.66 (3H, s), 3.65-3.45 (4H), 2.95 (2H, t, J=6.5Hz), 2.36 and 2.33 (each 3H, s).

| FORMULATION EXAMPLE 1 | |
|---|---|
| (1) Compound 2 | 10 g |
| (2) Fine particles for direct punching, No. 209 (produced by Fuji Kagaku K.K.; comprising 20% of magnesium metasilicate aluminate, 30% of corn starch and 50% of lactose | 110 g |
| (3) Crystalline cellulose | 60 g |
| (4) Calcium carboxymethyl cellulose | 18 g |
| (5) Magnesium stearate | 2 g |

Each of components (1), (3), and (4) was passed through a sieve of 100 mesh. Components (1), (3), (4), and (2) each was dried to have a prescribed water content and mixed in a mixing machine to form a homogeneous powder mixture. Component (5) was then added thereto, followed by mixing for 30 seconds. The resulting powder mixture was punched out to obtain tablets each weighing 200 mg.

If desired, the tablets may be coated with a commonly employed gastro-soluble film coating (e.g., polyvinylacetal diethylaminoacetate) or colorant for foodstuff.

| FORMULATION EXAMPLE 2 | |
|---|---|
| (1) Compound 2 | 50 g |
| (2) Lactose | 930 g |
| (3) Magnesium stearate | 20 g |

The above components were uniformly mixed, and the powder mixture was charged in hard gelatin capsules by 200 mg (total content).

| FORMULATION EXAMPLE 3 | |
|---|---|
| (1) Compound 2 | 5 mg |
| (2) Glucose | 100 mg |
| (3) Physiological saline | 10 mℓ |

A solution of a mixture of the above components was filtered and sterilized by bacterial filtration. The

filtrate was poured into a germ-free vial, and nitrogen gas was charged into the vial, followed by sealing to obtain an intravenous injection.

While the invention has been described in detail and with reference to specific embodiments thereof, it will be apparent to one skilled in the art that various changes and modifications can be made therein without departing from the spirit and scope thereof.

## Claims

1. A treating agent for angiospasm containing, as an active ingredient, an angiospasm treating effective amount of a dihydropyridine derivative represented by formula (I):

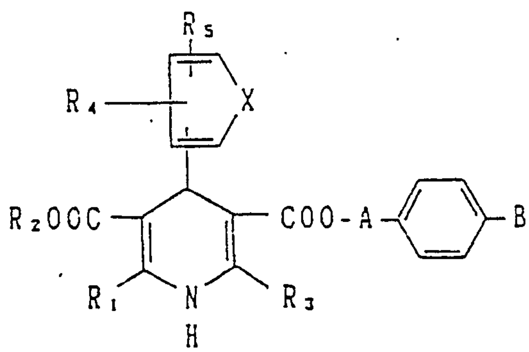

( I )

wherein $R_1$, $R_2$ and $R_3$ which may be the same or different, each represents an alkyl group or an alkoxyalkyl group; $R_4$ and $R_5$, which may be the same or different, each represents a hydrogen atom, a halogen atom, a nitro group, a halogenated alkyl group, an alkyl-sulfonyl group, a halogenated alkoxyl group, an alkylsulfinyl group, an alkyl group, an alkoxyl group, a cyano group, an alkoxycarbonyl group or an alkylthio group, provided that $R_4$ and $R_5$ do not simultaneously represent a hydrogen atom; X represents a vinylene group or an azomethine group; A represents an alkylene group; and B represents $-N(R_6)_2$ or

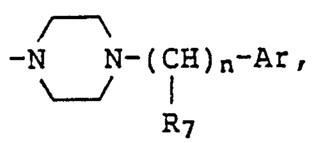

wherein $R_6$ and $R_7$ each represents a hydrogen atom, an alkyl group, an aralkyl group, an aryl group or a pyridyl group; Ar represents an aryl group or a pyridyl group; and n represents 0 or an integer of 1 or 2, or a pharmaceutically acceptable acid addition salt thereof and a pharmaceutically acceptable carrier.

2. A treating agent for angiospasm according to claim 1, wherein $R_1$, $R_2$ and $R_3$, which may be the same or different, each represents an alkyl group, $R_4$ represents a hydrogen atom, $R_5$ represents a nitro group, a halogenated alkyl group or a ciano group, $R_6$ represents an alkyl group, an aralkyl group or an aryl group, $R_7$ represents an aryl group, Ar represents an aryl group and n is 1.

3. A preventing agent for angiospasm containing, as an active ingredient, an angiospasm treating effective amount of a dihydropyridine derivative represented by formula (I):

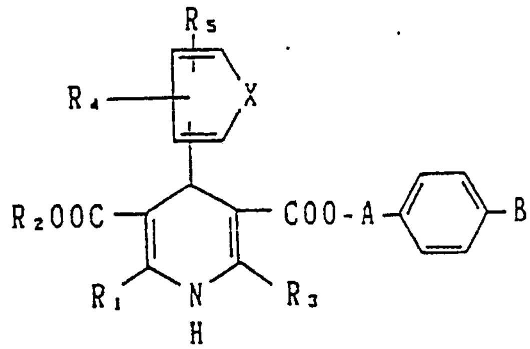

( I )

wherein $R_1$, $R_2$ and $R_3$ which may be the same or different, each represents an alkyl group or an alkoxyalkyl group; $R_4$ and $R_5$, which may be the same or different, each represents a hydrogen atom, a halogen atom, a nitro group, a halogenated alkyl group, an alkylsulfonyl group, a halogenated alkoxyl group, an alkylsulfinyl group, an alkyl group, an alkoxyl group, a cyano group, an alkoxycarbonyl group or an alkylthio group, provided that $R_4$ and $R_5$ do not simultaneously represent a hydrogen atom; X represents a vinylene group or an azomethine group; A represents an alkylene group; and B represents $-N(R_6)_2$ or

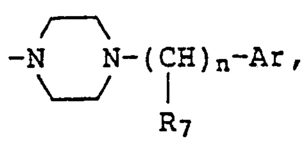

wherein $R_6$ and $R_7$ each represents a hydrogen atom, an alkyl group, an aralkyl group, an aryl group or a pyridyl group; Ar represents an aryl group or a pyridyl group; and n represents 0 or an integer of 1 or 2, or a pharmaceutically acceptable acid addition salt thereof and a pharmaceutically acceptable carrier.

4. A preventing agent for angiospasm according to claim 3, wherein $R_1$, $R_2$ and $R_3$, which may be the same or different, each represents an alkyl group, $R_4$ represents a hydrogen atom, $R_5$ represents a nitro group, a halogenated alkyl group or a ciano group, $R_6$ represents an alkyl group, an aralkyl group or an aryl group, $R_7$ represents an aryl group, Ar represents an aryl group and n is 1.

5. The use of a dihydropyridine derivative of claim 1 for the manufacture of a medicament for treating angiospasm.

6. The use of a dihydropyridine derivative of claim 1 for the manufacture of a medicament for preventing angiospasm.